Europäisches Patentamt

European Patent Office

Office européen des brevets

(19)

(11) Publication number: **0 056 741**

**B1**

(12) # EUROPEAN PATENT SPECIFICATION

(45) Date of publication of patent specification: **26.06.85**

(51) Int. Cl.⁴: **C 07 D 301/19, B 01 J 23/92**

(21) Application number: **82300319.9**

(22) Date of filing: **21.01.82**

(54) Epoxidation reaction with recovered molybdenum catalyst.

(30) Priority: **21.01.81 US 226969**

(43) Date of publication of application:
**28.07.82 Bulletin 82/30**

(45) Publication of the grant of the patent:
**26.06.85 Bulletin 85/26**

(84) Designated Contracting States:
**BE DE FR GB IT NL**

(56) References cited:
**GB-A-1 317 480**
**US-A-3 763 303**
**US-A-3 819 663**

(73) Proprietor: **ATLANTIC RICHFIELD COMPANY**
**Arco Plaza 515 S. Flower Street**
**Los Angeles California 90071 (US)**

(72) Inventor: **Taylor, Paul Duane**
**1128 Cymry Drive**
**Berwyn Pennsylvania 19312 (US)**
Inventor: **Mocella, Michael Thomas**
**308 Baywood Road**
**West Chester Pennsylvania 19380 (US)**

(74) Representative: **Cropp, John Anthony David**
**et al**
**MATHYS & SQUIRE 10 Fleet Street**
**London, EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Oxirane compounds such as ethylene oxide, propylene oxide, and their higher homologs are valuable articles of commerce. One of the most attractive processes for synthesis of those oxirane compounds is described by Kollar in United States Patent No. 3,351,635. According to Kollar, the oxirane compound (e.g., propylene oxide) may be prepared by expoxidation of an olefinically unsaturated compound (e.g., propylene) by use of an organic hydroperoxide and a suitable catalyst such as molybdenum.

During the epoxidation reaction the hydroperoxide is converted almost quantitatively to the corresponding alcohol. That alcohol may be recovered as a coproduct with the oxirane compound. However, it is the oxirane which is of primary concern.

Kollar teaches that oxirane compounds may be prepared from a wide variety of olefins. Lower olefins having three or four carbon atoms in an aliphatic chain are advantageously epoxidized by the process. The class of olefins commonly termed alpha olefins or primary olefins are epoxidized in a particularly efficient manner by the process. It is known to those in the art that primary olefins, e.g., propylene, butene-1, decene-1 hexadecene-1 etc., are much more difficulty epoxidized than other forms of olefins, excluding only ethylene. Other forms of olefins which are much more easily epoxidized are substituted olefins, alkenes with internal unsaturation, cycloalkenes and the like. Kollar teaches that notwithstanding the relative difficulty in epoxidizing primary olefins, epoxidation proceeds more efficiently when molybdenum, titanium or tungsten catalysts are used. Molybdenum is of special interest. Kollar teaches that activity of those metals for epoxidation of the primary olefins is surprisingly high and can lead to high selectivity of propylene to propylene oxide. These high selectivities are obtained at high conversions of hydroperoxide (50% or higher) which conversion levels are important for commercial utilization of the technology.

Kollar's epoxidation reaction proceeds under pressure in the liquid state and, accordingly, a liquid solution of the metal catalyst is preferred. Preparation of a suitable catalyst is taught by Sheng et al in United States Patent No. 3,434,975. According to Sheng, the reaction-medium soluble epoxidation catalyst may be prepared by reacting molybdenum metal with an organic hydroperoxide, per acid or hydrogen peroxide in the presence of a saturated alcohol having one to four carbon atoms.

When propylene is epoxidized with tertiary-butyl hydroperoxide according to the Kollar process using the Sheng catalyst, a product mixture containing unreacted propylene, propylene oxide, tertiary-butyl alcohol and molybdenum catalyst is obtained. Distillation of that product mixture provides substantially pure propylene oxide and tertiary-butyl alcohol. The residue of distillation (hereafter "TBA bottoms") contains spent molybdenum catalyst as well as high boiling organic residues.

Removal and recovery of the molybdenum values from the distillation residue are important from ecological and economical standpoints. In United States Patent No. 3,763,303 Khuri et al disclose two embodiments of a process for recovering molybdenum values from spent epoxidation catalysts. The Khuri process first embodiment involves recovery of molybdenum directly from the spent catalyst mixture by a liquid-to-liquid extraction utilizing an aqueous extractant consisting essentially of water which is intermittently admixed with the residue to be treated to effect an extraction and transfer of a portion of the molybdenum constituent from the organic phase to the aqueous phase. Untreated spent catalyst solutions usually contain molybdenum in concentrations of from about 0.1% to about 1.0% by weight and Khuri discloses those solutions are highly satisfactory for treatment in the liquid-to-liquid extraction process in which the extractant consists essentially of water to effect molybdenum separation. Molybdenum separated with the aqueous extract is recovered as molybdenum trioxide by evaporation of water followed by calcination of the solid obtained by extract evaporation.

The second embodiment of the Khuri process relates to extracting molybdenum from distillation residues obtained from distillation of spent catalyst solution (TBA bottoms) but the extraction is performed with acids or bases to convert the molybdenum into a recoverable molybdenum compound of the acid or base.

British Patent Specification 1,317,480 also teaches recovery of molybdenum values from spent epoxidation catalysts. As in Khuri, the British recovery process involves extracting the spent catalyst solution with water alone or with aqueous ammonia. The British extraction process results in a transfer of at least 95% of the available molybdenum values to the aqueous extract. Those molybdenum values are recovered from the aqueous phase by precipitation as a phosphomolybdate or by distillative stripping of the volatile organic material and water from the extract.

The spent catalyst solution may also be subjected to exhaustive evaporation or distillation to produce a residue with a higher molybdenum content as taught by Levine et al in United States Patent No. 3,819,663. The Levine process starts with a spent catalyst solution such as TBA bottoms and subject that solution to a wiped film evaporation at 375 to 450°F until 60 to 80% by weight of the solution is evaporated overhead. The residue of that evaporation is taught to be useful as a catalyst in further epoxidation processes.

It has now been discovered that a molybdenum solution suitable for use as an epoxidation catalyst can be obtained from spent epoxidation catalyst solutions without the molybdenum having to pass through an intermediate, solid phase.

In accordance with the invention, the spent catalyst solution is treated by

(a) extracting molybdenum values therefrom by a liquid-to-liquid extraction with an extracting medium consisting essentially of water or water and a water immiscible organic solvent,

(b) separating the molybdenum-rich aqueous phase, and

(c) removing water and lower boiling organic compounds from the separate aqueous phase extract to form a concentrated molybdenum solution.

and the concentrated molybdenum solution of (c) is introduced into a mixture of an olefin and a hydroperoxide to catalyse the epoxidation of the olefin.

The term "spent catalyst solution" as used herein is intended to mean that fraction of the epoxidation reaction product effluent remaining after removal of unreacted olefin (for example, propylene), alkylene oxide (for example, propylene oxide) and a major portion of the alcohol corresponding to the hydroperoxide (for example, tertiary butyl hydroperoxide) used in the epoxidation reaction which reaction may be according to the procedure of Kollar. Spent catalyst solution, apart from molybdenum compounds, contains some alcohol, acids and other low molecular weight oxygenated compounds and said spent catalyst solution is generally not subjected to any chemical treatment before being subjected to the process of the present invention. It is contemplated that spent catalyst solution as used herein includes both the distillation bottoms treated in British Patent Specification 1,317,480 and the residue obtained from the wiped film evaporation process according to Levine and said spent catalyst solution can contain molybdenum compounds at levels of up to 5% by weight.

By means of the invention molybdenum is recoverd from spent catalyst solution as an active, high quality catalyst suitable to catalyze a hydroperoxide oxidation of olefins.

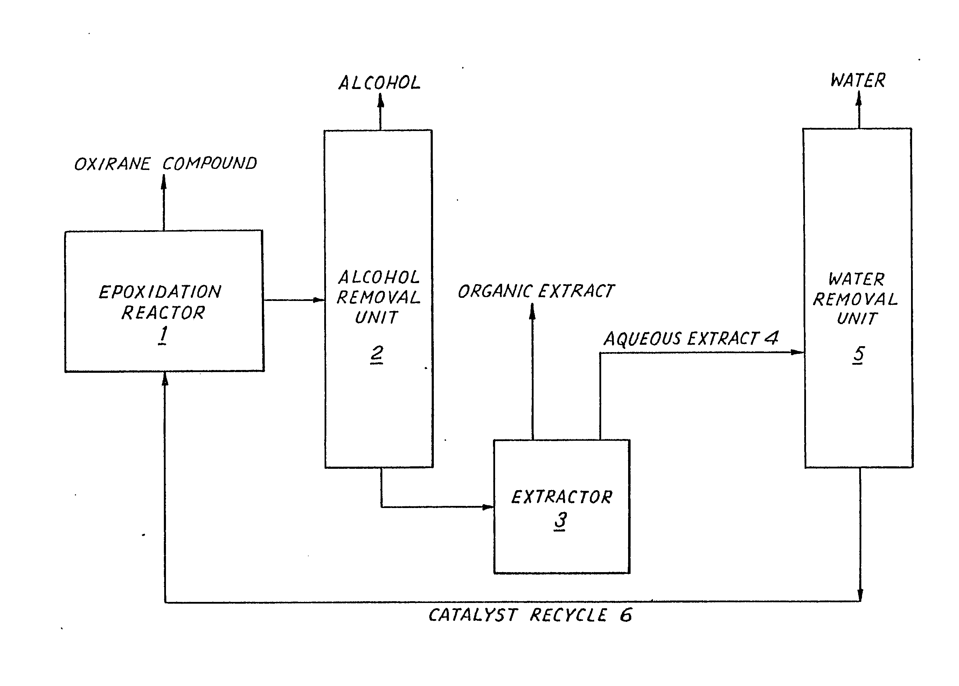

The invention will now be described in greater detail with reference to preferred embodiments and with the aid of the accompanying drawing which illustrates in flow chart fashion recovery and recycling of active molybdenum catalyst according to this invention.

It has now been discovered that an active molybdenum catalyst useful in hydroperoxide oxidations of olefins may be prepared from spent catalyst solutions obtained from a preceding epoxidation reaction. By use of the present process, the spent molybdenum catalyst can be recovered and reused without detrimental effects on the yields of epoxide product.

The epoxidation reaction from which a spent catalyst stream is recovered or into which may be recycled the active catalyst obtained by way of this invention is decribed by Kollar, discussed above. Both Kollar and the present invention are applicable to hydroperoxide epoxidation of a wide variety of olefins. The peroxides and olefins useful in the reaction which yields the spent catalyst stream herein are the same as those disclosed by Kollar. It is to be understood that by practice of the present invention, an active soluble molybdenum catalyst may be obtained from spent catalysts solutions obtained from crude reaction products of molybdenum-catalyzed epoxidation of any of the olefins by any of the peroxides disclosed by Kollar. Thus, the present invention is generically applicable to treatment of any spent catalyst solution as defined above. However, for the sake of simplicity and as a representative example only, the following description will concern epoxidation of propylene by tertiary-butyl hydroperoxide with a soluble molybdenum catalyst. But it should be understood the present invention is not limited to those species.

Referring now to the drawing, the figure illustrates in diagrammatic form the steps employed to obtain the recycled catalyst according to this invention. Epoxidation reactor 1 is pressure charged with tertiary-butyl hydroperoxide, propylene and soluble molybdenum catalyst prepared according to known methods. After epoxidation under the conditions taught by Kollar, product propylene oxide is removed from the reaction mixture leaving a by-product mixture containing, *inter alia*, spent catalyst solution and tertiary-butyl alcohol which is then passed into distillation column 2. Advantageously, valuable tertiary-butyl alcohol is distilled from the by-product mixture in column 2. The residue from column 2 ("TBA bottoms") contains molybdenum values and high molecular weight organic residues. In order to obtain a molybdenum catalyst of sufficient purity and activity to be suitably reused in further epoxidation processes, the molybdenum is desirably separated from most of the organic residues. Separation of the molybdenum values from the organic residue of spent catalyst solutions is of importance in producing a commercially attractive reusable catalyst. Simple molybdenum concentration and reuse of molybdenum values as taught by Levine, discussed above, is not entirely satisfactory because of the reintroduction of heavy organic residues into further epoxidations. Those heavy organic residues are believed to be responsible for the detrimental effect on commercial production of epoxide by lowering the epoxide yield when said concentrated molybdenum solutions are used.

Distillation residue from column 2 is passed to extractor 3 for liquid-to-liquid extraction for separation of molybdenum values from most of the organic residues. British 1,317,480 describes extracting distillation residues such as TBA bottoms with water or aqueous ammonia to transfer the molybdenum values to an aqueous phase. An improved extraction of spent catalyst solution is disclosed in our European patent publication No. 0056743. According to this publication, molybdenum may be extracted into water without an added acid or base when the TBA bottoms are extracted with water and a water-immiscible organic solvent for the organic residue. The aqueous extractions of TBA bottoms results in a two phase extraction system with one phase being aqueous and the other being organic. The aqueous extract solution phase contains mostly water but also along with dissolved molybdenum values, the aqueous phase may contain low molecular weight organic material from the spent catalyst solution. Higher weight molecular weight organic materials remain in the organic phase extract.

When TBA bottoms are subjected to aqueous extraction in extractor 3 according to the British procedure or according to our above-described improved extraction process, it has been discovered that an active molybdenum catalyst can be produced directly without solids isolation from the aqueous extract 4. To produce the dissolved molybdenum catalyst useful in epoxidation reactor 1 from aqueous extract 4, extract 4 is passed to distillation column 5 wherein water and lower molecular weight organic materials are stripped off. According to this invention an active recycled molybdenum catalyst is obtained by passing aqueous extract 4 to distillation column 5. Extract 4 is distilled to remove water at atmospheric pressure or at pressures lower than atmospheric. Distillation pot temperatures of up to about 200°C may be employed but when the distillation residue temperature exceeds 120°C the distillation environment must be such that there is minimal refluxing and vapour is removed from the distill and as fast as such vapor forms. It is most preferred to keep the distillation pot temperature below 120°C and, accordingly, reduced pressure in the distillation may be used to advantage. Distillation should be continued until all compounds are distilled having a boiling point of at least 100°C at standard pressure. Optionally, additionally distillation may be performed to remove these compounds boiling at 100°C to 200°C at standard pressure but removal of still higher boiling material is of limiting benefit. The bottoms (residue) from distillation column 5 is a homogeneous liquid with no solid residue. The liquid bottoms of distillation column 5 is a high quality liquid molybdenum catalyst which is suitable for direct reuse in epoxidation reactor 1. Reuse of the regenerated liquid catalyst may be direct recycle as shown by recycle line 6 of the Figure or by reuse in another separate epoxidation reaction. The reusable liquid molybdenum catalyst can contain up to about 5% by weight of dissolved molybdenum compounds.

In order to further illustrate the subject matter of the present invention, the following examples are provided. However, it is to be understood that the examples are merely illustrative and are not intended as being restrictive of the invention herein disclosed and as defined by the annexed claims. Parts and percentages are by weight and temperatures are given in degrees centigrade unless otherwise specified.

## Example 1

A 200 part portion of crude TBA bottoms was extracted with an equal weight of water for 30 minutes at 95°. One hundred parts of the resulting aqueous extract was distilled at atmospheric pressure until a final pot temperature of 175° was reached. Nineteen parts of liquid material remained undistilled. This liquid contained no solid residue, and contained 13,850 ppm soluble molybdenum and 1.9% water. The molybdenum-containing liquid residue was an effective catalyst for the epoxidation of propylene with tertiary-butyl hydroperoxide.

## Example 2

A 75 part portion of crude TBA bottoms was extracted twice with equal weights of water at 95° for 30 minutes. A 150 part portion of the resulting aqueous extract, containing 2000 ppm molybdenum and 72% water was then flash evaporated at 95% under a pressure of 3 mm Hg. The undistilled residue of 8.6 parts contained 37,000 ppm soluble molybdenum and less than 0.2% water. The molybdenum-containing liquid residue was an effective catalyst for the epoxidation of propylene with tertiary-butyl hydroperoxide.

## Example 3

One hundred parts each of distillation bottoms and water were contacted for 30 minutes at 100°. The cooled aqueous phase of 138 parts was then concentrated by vacuum distillation at a pressure of 4 mm Hg until a distillation pot temperature of 100° was reached. The pot residue consisted of 12 parts of a dark, moderately viscous liquid containing 34,000 ppm molybdenum.

A stainless steel autoclave is charged with 70 parts of propylene and heated to 132°. At that point, a mixture of 20 parts tertiary-butyl hydroperoxide and 50 parts tertiary-butanol which contains 100 ppm molybdenum derived from the above concentration process is added to the reactor. After 30 minutes reaction 130°, the mixture is quenched and analyzed for propylene oxide and unreacted hydroperoxide. The tertiary-butyl hydroperoxide reacted amounts to 78% of that charged, and propylene oxide is produced in 92% selectivity versus hydroperoxide reacted.

## Example 4

One hundred parts each of distillation bottoms, No. 2 fuel oil, and water were well mixed for 15 minutes at 170°. The cooled aqueous phase which amounted to 146 parts was then concentrated by vacuum distillation at a reduced pressure of 4 mm Hg until distillation pot temperature of 100° was reached. The 35.5 parts of dark, moderately viscous pot residue contained 28,300 ppm molybdenum.

The molybdenum-containing solution is then used as a propylene epoxidation catalyst as in Example 3. After 90 minutes reaction, 96% of the charged hydroperoxide had reacted, and propylene oxide was produced in 88% selectivity versus hydroperoxide reacted.

**Claims**

1. A process of epoxidation of an olefinic compound with an organic hydroperoxide catalysed by a liquid solution of dissolved molybdenum wherein the fraction of the crude epoxidation product reaction mixture remaining after treatment to remove unreacted olefin, product olefin oxide and a major portion of the alcohol corresponding to the hydroperoxide reactant, is treated by

(a) extracting molybdenum values therefrom by a liquid-to-liquid extraction with an extracting medium consisting essentially of water or water and a water immiscible organic solvent,

(b) separating the molybdenum-rich aqueous phase,

(c) removing water and lower boiling organic compounds from the separated aqueous phase extract to form a concentrated molybdenum solution,

characterised in that the concentrated molybdenum solution of (c) is introduced into a mixture of an olefin and a hydroperoxide to catalyze the epoxidation of the olefin.

2. The process according to claim 1 wherein the concentrated molybdenum solution is recycled into a subsequent epoxidation reaction of the type from which it is obtained.

3. The process according to claim 1 wherein the concentrated molybdenum solution is used in another, different epoxidation reaction from which it is obtained.  –

4. The process according to any one of claims 1 to 3 wherein the concentrated molybdenum solution of (c) is obtained by removing water and organic compounds which boil below 200°C at standard pressure.

5. The process according to claim 4 wherein the concentrated molybdenum solution is obtained by distillation at a reduced pressure to a final bottom temperature of up to 120°C.

**Patentansprüche**

1. Verfahren zur Epoxidation einer Olefinverbindung mit einem organischen Hydroperoxid, die durch eine flüssige Lösung von gelöstem Molybdän katalysiert wird, worin die Fraktion des rohen Epoxidationsprodukt-Reaktionsgemisches, die nach der Behandlung zurückbleibt, zur Entfernung von nicht umgesetztem Olefin, Produkt-Olefinoxid und eines Hauptteils des Alkohols, der dem Hydroperoxid-Reaktanten entspricht, behandelt wird durch

(a) Extraktion von Molybdänwerten daraus durch eine Flüssigkeits-Flüssigkeits-Extraktion mit einem Extraktionmedium, welches in wesentlichen aus Wasser oder aus Wasser und einem mit Wasser nicht mischbaren organischen Lösungsmittel besteht,

(b) Abtrennung der Molybdän-reichen wässrigen Phase,

(c) Entfernung des Wassers und organischer Verbindungen mit niedrigen Siedepunkten von dem abgetrennten Wässrigphasen-Extrakt, um eine konzentrierte Molybdän-Lösung zu bilden,

dadurch gekennzeichnet, daß die konzentrierte Molybdän-Lösung von (c) in ein Gemisch eines Olefins und eines Hydroperoxids eingeführt wird, um die Epoxidation des Olefins zu katalysieren.

2. Verfahren nach Anspruch 1, worin die konzentrierte Molybdän-Lösung zurückgeführt wird in eine anschliessende Epoxidationsreaktion des Typs, aus dem sie erhalten wurde.

3. Verfahren nach Anspruch 1, worin die konzentrierte Molybdän-Lösung in einer anderen unterschiedlichen Epoxidationsreaktion, als derjenigen, aus der sie erhalten wurde, verwendet wird.

4. Verfahren nach einem der Ansprüche 1 bis 3, worin die konzentrierte Molybdän-Lösung von (c) durch Entfernung von Wasser und organischer Verbindungen, die unterhalb von 200°C bei Standarddruck sieden, erhalten wird.

5. Verfahren nach Anspruch 4, worin die konzentrierte Molybdän-Lösung durch Destillation unter vermindertem Druck zu einer endgültigen Bodentemperatur von bis zu 120°C erhalten wird.

**Revendications**

1. Procédé d'époxydation d'un composé oléfinique avec un hydroperoxyde organique, catalysé par une solution liquide de molybdène dissous, selon lequel la fraction du mélange réactionnel brut résultant de l'époxydation et restant après le traitement éliminant l'oléfine n'ayant pas réagi, l'oxyde d'oléfine obtenu et une portion majeure de l'alcool correspondant à l'hydroperoxyde réactif, est traitée de la façon suivante:

(a) on en extrait le entités de molybdène en cours d'une extraction liquide-liquide avec un milieu d'extraction consistant essentiellement en eau ou en eau et en solvant organique nonmiscible à l'eau,

(b) on sépare la phase aqueuse riche en molybdène,

(c) on sépare l'eau et des composés organiques à point d'ébullition inférieur à partir de la phase aqueuse extraite séparée pour former une solution concentrée en molybdène,

caractérisé en ce que la solution concentrée en molybdène de (c) est introduite dans un mélange d'une oléfine et d'un hydroperoxyde, pour catalyser l'époxydation de l'oléfine.

2. Procédé selon la revendication 1, caractérisé en ce que la solution concentrée en molybdène est recyclée dans une réaction d'époxydation ultérieure, du type à partir duquel elle est obtenue.

3. Procédé selon la revendication 1, caractérisé en ce que la solution concentrée en molybdène est utilisée dans une autre réaction d'époxydation, différente de celle à partir de laquelle elle est obtenue.

**4.** Procédé selon l'une quelconque des revendications 1 à 3, caractérisé en ce que l'on obtient la solution concentrée en molybdène de (c) en enlevant l'eau et des composés organiques qui bouillent en dessous de 200°C à la pression standard.

**5.** Procédé selon la revendication 4, caractérisé en ce que l'on obtient la solution concentrée en molybdène, en distillant sous pression réduite jusqu'à une température finale de queue allant jusqu'à 120°C.

OXIRANE COMPOUND

ALCOHOL

WATER

EPOXIDATION REACTOR _1_

ALCOHOL REMOVAL UNIT _2_

ORGANIC EXTRACT

AQUEOUS EXTRACT 4

WATER REMOVAL UNIT _5_

EXTRACTOR _3_

CATALYST RECYCLE 6